# EUROPEAN PATENT APPLICATION

(11) **EP 4 512 255 A1**
(43) Date of publication of application: **26.02.2025**
(21) Application number: 23791882.6
(22) Date of filing: 19.04.2023
(51) Int. Cl.: A23L 33/135

(54) **ANTI-HUMAN CORONAVIRUS COMPOSITION**

(30) Priority: 20.04.2022 JP 2022069288
(71) Applicant: Meiji Co., Ltd., Chuo-ku Tokyo 104-8306 (JP)
(72) Inventor: TANG, Shuyi, Hachioji-shi, Tokyo 192-0919 (JP); ISHII, Shoko, Hachioji-shi, Tokyo 192-0919 (JP); OGAWA, Miho, Hachioji-shi, Tokyo 192-0919 (JP)
(74) Representative: TBK
(86) International application number: PCT/JP2023/015554
(87) International publication number: WO 2023/204234

(57) **Abstract**

An objective of the present invention is to provide a nonpharmaceutical anti-human coronavirus composition that prevents human coronavirus infections, the onset of the infections, or aggravation of symptoms of the infections. The anti-human coronavirus composition according to the present invention includes an exopolysaccharide produced by a lactic acid bacterium in genus *Lactobacillus* as an active component. The lactic acid bacterium in the genus *Lactobacillus* may belong to a *Lactobacillus delbrueckii* species. The lactic acid bacterium in the genus *Lactobacillus* may specifically be *Lactobacillus delbrueckii subsp. bulgaricus* OLL1073R-1 (FERM BP-10741).

## Description

### TECHNICAL FIELD

The present invention relates to an anti-human coronavirus composition.

### BACKGROUND ART

Human coronaviruses (HCoVs) are widely known as pathogens of the common cold. Examples of HCoVs include HCoV-229E, HCoV-OC43, HCoV-NL63, and HCoV-HKU1, and MERS-CoV, SARS-CoV, and SARS-CoV2 causing severe pneumonia. HCoV-229E, HCoV-OC43, HCoV-NL63, and HCoV-HKU1 cause 10 to 15% of the common cold (about 35% in epidemic season), and the epidemics peak mainly in winter.

### PRIOR ART DOCUMENT

### PATENT DOCUMENT

Patent Document 1: Japanese Unexamined Patent Application Publication No. 2005-194259
Patent Document 2: Japanese Unexamined Patent Application Publication No. 2005-247757
Patent Document 3: Japanese Unexamined Patent Application Publication (Translation of PCT Application) No. 2011-512319
Patent Document 4: Japanese Unexamined Patent Application Publication No. 2012-038451
Patent Document 5: Japanese Unexamined Patent Application Publication No. 2018-177740
Patent Document 6: Japanese Unexamined Patent Application Publication No. 2021-080187

### SUMMARY OF THE INVENTION

### PROBLEMS TO BE SOLVED BY THE INVENTION

Methods with minimum use of pharmaceuticals to prevent HCoV infections, the onset of the infections, or aggravation of symptoms of the infections have been awaited recently. One or more aspects of the present invention are directed to provide a nonpharmaceutical anti-HCoV composition that prevents HCoV infections, the onset of the infections, or aggravation of symptoms of the infections.

### MEANS FOR SOLVING THE PROBLEMS

In response to the above issue, the inventors have focused on exopolysaccharides (nonpharmaceutical substances) produced by lactic acid bacteria in the genus *Lactobacillus,* and examined the anti-human coronavirus effects of the exopolysaccharides in detail. As a result of the examination, the inventors have noticed anti-HCoV effects of an intake of the exopolysaccharides and developed aspects of the present invention.

One or more aspects of the present invention are described below.
(1) An anti-human coronavirus composition, comprising:
   an exopolysaccharide produced by a lactic acid bacterium in genus *Lactobacillus* as an active component. The anti-human coronavirus composition may consist of the exopolysaccharide or may contain the exopolysaccharide and other components.
(2) The anti-human coronavirus composition according to (1), wherein the anti-human coronavirus composition is a composition to reduce proliferation of a human coronavirus.
(3) The anti-human coronavirus composition according to (2), wherein the composition is a composition to reduce proliferation of a human coronavirus in non-immune cells.
(4) The anti-human coronavirus composition according to (3), wherein the non-immune cells are lung cells.
(5) The anti-human coronavirus composition according to (1), wherein the anti-human coronavirus composition is a preventive composition to prevent at least one selected from the group consisting of (a) a human coronavirus infection, (b) an onset of the human coronavirus infectious disease, and (c) aggravation of a symptom attributable to the human coronavirus infection.
(6) The anti-human coronavirus composition according to (1), wherein the anti-human coronavirus composition is a composition for quick recovery from an onset of a human coronavirus infection.
(7) The anti-human coronavirus composition according to (1), wherein the human coronavirus is a respiratory infection human coronavirus.
(8) The anti-human coronavirus composition according to (1), wherein the lactic acid bacterium in the genus *Lactobacillus belongs* to a *Lactobacillus delbrueckii* species.
(9) The anti-human coronavirus composition according to (8), wherein the lactic acid bacterium in the genus *Lactobacillus* is *Lactobacillus delbrueckii subsp. bulgaricus* OLL1073R-1 (FERM BP-10741).
   One or more aspects of the present invention are also directed to the aspects described below.
(10) A method for improving resistance (immune strength) against a human coronavirus, comprising: administering a composition containing an exopolysaccharide produced by a lactic acid bacterium in genus *Lactobacillus* to a subject in need thereof.
(11) A method for improving resistance (immune strength) against a human coronavirus, comprising: administering an exopolysaccharide produced by a lactic acid bacterium in genus *Lactobacillus* to a subject.
(12) A method comprising: using an exopolysaccharide produced by a lactic acid bacterium in genus *Lactobacillus* as an anti-human coronavirus agent.
(13) Use of an exopolysaccharide produced by a lactic acid bacterium in genus *Lactobacillus* to manufacture an anti-human coronavirus composition.
(14) Use of an exopolysaccharide produced by a lactic acid bacterium in genus *Lactobacillus* in manufacture of an anti-human coronavirus composition.
(15) An exopolysaccharide produced by a lactic acid bacterium in genus *Lactobacillus,* the exopolysaccharide being usable in manufacture of an anti-human coronavirus composition.
(16) An exopolysaccharide produced by a lactic acid bacterium in genus *Lactobacillus,* the exopolysaccharide being usable as an anti-human coronavirus agent.
(17) A method for manufacturing an anti-human coronavirus composition, comprising: supplying a dairy ingredient to a lactic acid bacterium in genus *Lactobacillus.*

### EFFECTS OF THE INVENTION

In the present invention, an intake of an exopolysaccharide (a nonpharmaceutical product) produced from a lactic acid bacterium in the genus *Lactobacillus* prevents HCoV infections, the onset of the infections, or aggravation of symptoms of the infections.

### BRIEF DESCRIPTION OF THE DRAWINGS

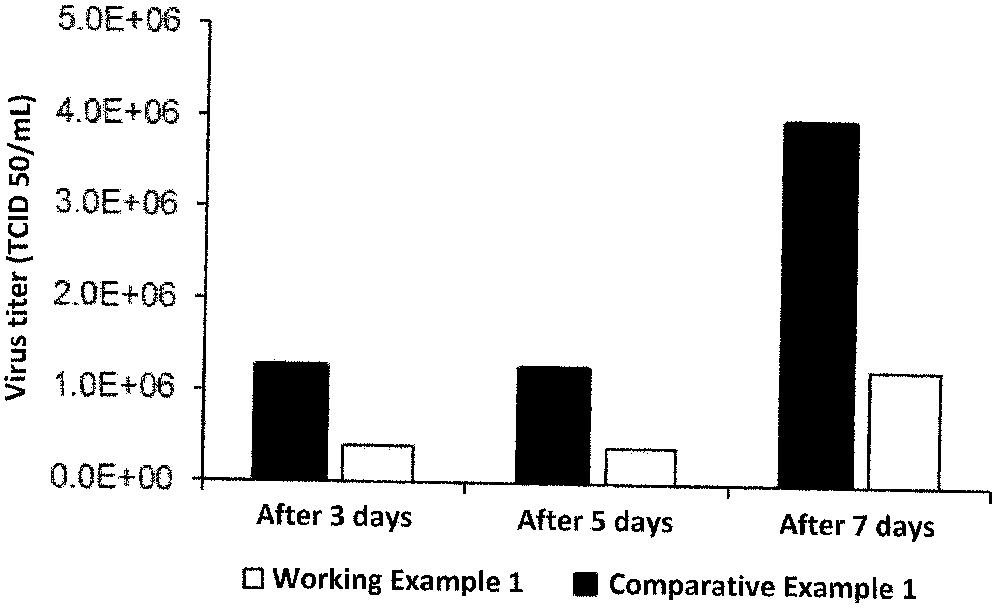

[FIG. 1] FIG. 1 is a bar graph showing virus titers of HCoV (HCoV-229E) infecting the human fetal lung fibroblast strain MRC-5 three, five, and seven days after the infection in Working Example 1 and Comparative Example 1.

### DETAILED DESCRIPTION OF THE INVENTION

An anti-human coronavirus (HCoV) composition according to one or more embodiments of the present invention contains an exopolysaccharide produced from a lactic acid bacterium in the genus *Lactobacillus.* The exopolysaccharide may be produced from *Lactobacillus delbrueckii,* specifically from *Lactobacillus delbrueckii subsp. bulgaricus,* or more specifically from *Lactobacillus delbrueckii subsp. bulgaricus* OLL1073R-1 (deposit number: FERM BP-10741). Although these exopolysaccharides may be any exopolysaccharides with intended effects, they may be exopolysaccharides produced by fermenting milk. One exopolysaccharide or a combination of two or more exopolysaccharides may be used in one or more embodiments of the present invention.

The exopolysaccharide functions as an anti-HCoV active component. The exopolysaccharide contains a phosphorylated polysaccharide. The exopolysaccharide may contain a nonphosphorylated polysaccharide that does not contain a sulfonated polysaccharide. This anti-HCoV composition is particularly effective against respiratory infection HCoVs. HCoVs are enveloped, negative-stranded RNA viruses and mainly infect the upper respiratory tract. Coronaviruses are classified into the family Coronaviridae in the order Nidovirales in virus taxonomy. The family Coronaviridae is specifically an envelope virus with the longest genome size in RNA viruses, and encodes 16 nonstructural proteins and 4 structural proteins in the gene as a basic genetic structure skeleton of a coronavirus. The structural proteins include spike, envelope, matrix, and nucleocapsid to form a virus particle, and contain a virus genome of 30 kb in a virus particle with a diameter of 80 to 160 nm (Reference Document 1).
Reference Document 1: Wataru Kamitani, Gunma University, Graduate School of Medicine, Faculty of Medicine, Department of Infectious Diseases and Host Defense, 1. The Basis of Coronavirus. Virus, 2020, vol. 70, No. 1, 29-36.

The anti-HCoV composition may be simply formed from an exopolysaccharide, or may contain an exopolysaccharide and another component.

Examples of a method for preparing the anti-HCoV composition include (a) a method for culturing a lactic acid bacterium in the genus *Lactobacillus* on a dairy medium, and obtaining the dairy culture as an anti-HCoV composition, (b) a method for adding the dried or condensed dairy culture described above to, for example, food to obtain an anti-HCoV composition, (c) a method for extracting an exopolysaccharide from the above dairy culture to use the exopolysaccharide as an anti-HCoV composition, and (d) a method for preparing fermented milk using a lactic acid bacterium in the genus *Lactobacillus* to use the fermented milk as an anti-HCoV composition.

Preparing the anti-HCoV composition as fermented milk facilitates an intake of the anti-HCoV composition as food or drink. Fermented milk has been taken for a long time and has a high level of safety without any concern of side effects. Thus, consumers can safely take the anti-HCoV composition. An example method for preparing fermented milk includes a method including sterilizing and cooling material milk, adding a lactic acid bacterium starter containing a lactic acid bacterium in the genus *Lactobacillus* described above to the material milk, and fermenting the material milk containing the lactic acid bacterium starter at a temperature and time to have a predetermined acidity. The exopolysaccharide is produced by the lactic acid bacterium in the genus *Lactobacillus* during ferment. With this method, the lactic acid acidity is for example 0.6 to 1.2. The lactic acid acidity may be 0.6 to 0.8. The ferment temperature is, for example, 40 to 45 °C. The ferment time is, for example, 2 to 12 hours. The ferment time may be 3 to 8 hours.

In one or more embodiments of the present invention, the anti-HCoV composition can reduce HCoV proliferation in non-immune cells, and thus can also be referred to as a composition reducing HCoV proliferation in non-immune cells. Non-immune cells are, for example, lung cells.

In one or more embodiments of the present invention, the anti-HCoV composition can (a) prevent HCoV infections, (b) prevent the onset of HCoV infections, or (c) prevent aggravation of symptoms attributable to HCoV infections. Thus, the anti-HCoV composition can also be referred to as an HCoV infection preventive composition, an HCoV infectious disease onset preventive composition, or an HCoV infectious disease aggravation preventive composition.

In one or more embodiments of the present invention, an anti-HCoV composition allows quick recovery from the onset of HCoV infectious disease, and thus can be referred to as a composition for quick recovery from HCoV infection onset or a composition for promoting recovery from HCoV infectious disease.

Lactic acid bacteria in the genus *Lactobacillus* in the present invention are a generic name of bacteria certified as lactic acid bacteria of the genus *Lactobacillus* in taxonomy, and are not limited to a specific species or strains. Lactic acid bacteria in the genus *Lactobacillus* may be classified by a plant-derived group and an animal-derived group, but either plant-derived lactic acid bacteria or animal-derived lactic acid bacteria are usable in the present invention. The strains of lactic acid bacteria in the genus *Lactobacillus* in the present invention may be one or more strains belonging to lactic acid bacteria in the genus *Lactobacillus* usable to prepare fermented milk (e.g., yogurt) taken for a long time. Lactic acid bacteria in the genus *Lactobacillus* in one or more embodiments of present invention may belong to *Lactobacillus delbrueckii* or *Lactobacillus delbrueckii subsp. bulgaricus,* or specifically be *Lactobacillus delbrueckii subsp. bulgaricus* OLL1073R-1 (deposit number: FERM BP-10741) or a derivative strain or a mutant strain of *Lactobacillus delbrueckii subsp. bulgaricus* OLL1073R-1.

The sequence number 1 in the sequence table below indicates the base sequence (also referred to as a nucleotide sequence) of the sequence in V1 to V3 regions of 16S rRNA gene (corresponding to the 34th to 535th base sequences in the full sequence of 16S rRNA gene) of *Lactobacillus delbrueckii subsp. bulgaricus* OLL 1073R-1.

### (Sequence Table)

| |
|---|
| <16S rRNA Gene (Sequence Number 1)> |
| GCTGGCGGCGTGCCTAATACATGCAAGTCGAGCGAGCTGAATTC AAAGATYCCTTCGGGRTGATTTGTTGGACGCTAGCGGCGGATGG GTGAGTAACACGTGGGCAATCTGCCCTAAAGACTGGGATACCACT TGGAAACAGGTGCTAATACCGGATAACAACATGAATCGCATGATT CAAGTTTGAAAGGCGGCGYAAGCTGTCACTTTAGGATGAGCCCG CGGCGCATTAGCTAGTTGGTGGGGTAAAGGCCTACCAAGGCAAT GATGCGTAGCCGAGTTGAGAGACTGATCGGCCACATTGGGACTG AGACACGGCCCAAACTCCTACGGGAGGCAGCAGTAGGGAATCTT CCACAATGGACGCAAGTCTGATGGAGCAACGCCGCGTGAGTGAA GAAGGTTTTCGGATCGTAAAGCTCTGTTGTTGGTGAAGAAGGATA GAGGCAGTAACTGGTCTTTATTTGACGGTAATCAACCAGAAAGTC ACGGCTAACTACGT |

Lactic acid bacteria in the genus *Lactobacillus* in the present invention may have productivity of polysaccharides. The polysaccharides may include galactose and glucose as constituent sugars, and contain phosphorus. Examples of lactic acid bacteria in the genus *Lactobacillus* of this type may have a 16S rRNA gene having the sequence identity greater than or equal to 95% with the base sequence of the sequence number 1 in the above sequence table (more specifically, the entire sequence of the 16S rRNA gene or a characteristic portion of the sequence, such as region V1, region V2, region V3, entire or part of regions V1, V2, and V3, or a portion including regions V1, V2, and V3), specifically have a 16S rRNA gene having the sequence identity greater than or equal to 98% with the base sequence of the sequence number 1, or more specifically have a 16S rRNA gene having the sequence identity greater than or equal to 99% with the base sequence of the sequence number 1.

Strains having the same mycological characteristics as *Lactobacillus delbrueckii subsp. bulgaricus* OLL1073R-1 are usable. The strains have mycological characteristics described below.

### (1) Morphological Characteristics

Shape of cells: Bacillus
Motility: None
Spore: None
Gram staining: Positive

### (2) Growth on Medium

The strains are applied to a plate of a blood liver (BL) agar medium (EIKEN CHEMICAL CO., LTD.), are cultured for 48 hours at 37 °C with a steel wool method, and have opaque rough colony morphology.

### (3) Physiological Characteristics

Nitrate reduction: Negative
Indologenous: Negative
Gelatin liquefaction: Negative
Catalase: Negative
Reaction to oxygen: Facultative Anaerobe
   D(-) lactic acid is produced from glucose with homolactic fermentation, and grown on a BL liquid medium not producing a gas, with negative growth at 10 °C, and positive growth at 45 °C.
Arginine Degradability: Negative
Gas Production from Malic Acid: Negative

### Degradability of Various Carbohydrates (Positive +, Negative -)

| | |
|---|---|
| Arabinose | - |
| Xylose | - |
| Rhamnose | - |
| Ribose | - |
| Glucose | + |
| Mannose | + |
| Fructose | + |
| Galactose | - |
| Sucrose | - |
| Maltose | - |
| Cellobiose | - |
| Lactose | + |
| Trehalose | - |
| Melibiose | - |
| Raffinose | - |
| Melezitose | - |
| Dextrin | - |
| Starch | - |
| Glycogen | - |
| Inulin | - |
| Mannitol | - |
| Sorbitol | - |
| Inositol | - |
| Esculin | - |
| Salicin | - |

### (4) Polysaccharide Productivity

As described above, *Lactobacillus delbrueckii subsp. bulgaricus* OLL1073R-1 has polysaccharide productivity, and extracellularly produces polysaccharides including galactose and glucose as constituent sugars and containing phosphorus.

As a lactic acid bacterium starter, lactic acid bacteria in the genus *Streptococcus* or *Streptococcus thermophilus* may be used together with lactic acid bacteria in the genus *Lactobacillus* to enhance production efficiency and palatability. When *Lactobacillus delbrueckii subsp. bulgaricus* OLL1073R-1 (deposit number: FERM BP-10741) is used as a lactic acid bacterium in the genus *Lactobacillus, Streptococcus thermophilus* 1131 or *Streptococcus thermophilus* OLS3059 may be used as a thermophilus. For use as a lactic acid bacterium starter or to provide, for example, functionality, a lactic acid bacterium other than lactic acid bacteria in the genus *Lactobacillus* or *Streptococcus* lactic acid bacteria or a microzyme such as *Lactococcus, bifidobacterium,* or yeast may be added.

*Lactobacillus delbrueckii subsp. bulgaricus* OLL1073R-1 is deposited in Patent Organism Depositary in National Institute of Technology and Evaluation (2-5-8-120, Kazusakamatari, Kisarazu-shi, Chiba, Japan) at the deposit number of FERM BP-10741 (deposit date: November 29, 2006). *Streptococcus thermophilusOLS3059* is deposited in Patent Organism Depositary in National Institute of Technology and Evaluation (2-5-8-120, Kazusakamatari, Kisarazu-shi, Chiba, Japan) at the deposit number of FERM BP-10740 (deposit date: November 29, 2006). *Streptococcus thermophilus* 1131 is available at LB81 Meiji Bulgaria Yogurt from Meiji Co., Ltd.

Fermented milk in one or more embodiments of the present invention is prepared by fermenting milk. Fermented milk may be, for example, fermented milk, lactic acid bacteria drink, lactic drink, or natural cheese defined by the Ministerial Ordinance on Milk and Milk Products Concerning Compositional Standards (the Ministerial Ordinance on Milk and Milk Products), but is not limited to these. For example, fermented milk may be fermented milk defined by the Ministerial Ordinance on Milk and Milk Products, or more specifically, obtained by fermenting, with lactic acid bacteria or yeasts, raw milk, milk, special milk, raw goat milk, sterilized goat milk, raw sheep milk, homogenized milk, low-fat milk, nonfat milk, processed milk, or milk containing an equivalent nonfat milk solid content or more into a solid state (hard state), a paste-like state (soft state), a liquid state (drink state), or a frozen state. Fermented milk is not limited to the above examples. Fermented milk in one or more embodiments of the present invention may have the concentration of a nonfat milk solid content within a range of, for example, 4.0 to 12.0% inclusive by mass, specifically 6.0 to 10.0% inclusive by mass, or more specifically 7.0 to 9.0% inclusive by mass. The concentration of the butterfat content may be within a range of, for example, 0.2 to 4.0% inclusive by mass, specifically 0.3 to 3.0% inclusive by mass, or more specifically 0.4 to 2.0% inclusive by mass.

In one or more embodiments of the present invention, a daily intake of exopolysaccharides may be greater than or equal to 100 pg, specifically greater than or equal to 500 pg, more specifically greater than or equal to 1.0 mg, or still more specifically greater than or equal to 2.0 mg. The upper limit of a daily intake of exopolysaccharides is not limited to a particular value, but may be, for example, 200 mg, specifically 100 mg, more specifically 70 mg, more specifically 35 mg, more specifically 30 mg, or still more specifically 8.0 mg.

Typical examples of fermented milk include yogurt. The international standards defined by Food and Agriculture Organization of the United Nations (FAO) or the World Health Organization (WHO) define that a product named yogurt is produced from a dairy product such as milk or powdered skim milk with lactic acid fermentation of *Streptococcus thermophilus* and *Lactobacillus delbrueckii ssp. bulgaricus,* and the above two bacteria are abundant and viable in the resultant product. In the present invention, yogurt includes yogurt defined by FAO/WHO as described above. This type of yogurt includes, for example, plain yogurt, hard yogurt (set yogurt), soft yogurt, and yogurt drink. In the present invention, yogurt drink may be used for ease of intake.

For convenience, an anti-HCoV composition according to one or more embodiments of the present invention may be individually packaged in an amount appropriate for each intake to facilitate appropriate intake of the anti-HCoV composition according to the embodiments of the present invention. The amount appropriate for each intake depends on the degree of infections or symptoms, and varies between individuals. However, for the anti-HCoV composition formed from fermented milk with, for example, a nonfat milk solid content of 8.0% by mass, the amount appropriate for each intake may be within a range of 10 to 1000 mL inclusive, specifically 30 to 500 mL inclusive, more specifically 50 to 200 mL inclusive, or still more specifically 80 to 120 mL inclusive. In some embodiments, the amount appropriate for each intake may be within a range of 10 to 1000 g inclusive, 30 to 500 g inclusive, 50 to 200 g, specifically 80 to 150 g inclusive, or more specifically 100 to 120 g inclusive.

In one or more embodiments of the present invention, an individual package may be any general package, for example, a lidded container, a capped bottle, an individual bag, a pouch, or a tube. The use purpose of the anti-HCoV composition according to one or more embodiments of the present invention may be clarified by at least one of describing, for example, the use purpose, effects, and the intake method for the anti-HCoV composition according to one or more embodiments of the present invention on an individual package or a package containing multiple individual packages, by enclosing, for example, the information in the individual package or the package containing multiple individual packages, or by separately providing the information to consumers with, for example, pamphlets.

To enhance the anti-HCoV effect, the anti-HCoV composition according to one or more embodiments of the present invention may be successively taken for three days or more, five days or more, a week or more, two weeks or more, four weeks or more, specifically six weeks or more, more specifically eight weeks or more, ten weeks or more, twelve weeks or more, 24 weeks or more, or still more specifically 36 weeks or more. For an anti-HCoV composition according to one or more embodiments of the present invention formed from fermented milk, the anti-HCoV composition has been taken for a long time and thus can be taken safely. The upper limit of the intake period is thus not limited to a particular period and the intake may be permanently continued. The upper limit may be, for example, 60 weeks or less. This upper limit may vary depending on the degree of infections or symptoms, for example, may be 120 weeks or less, 100 weeks or less, or 80 weeks or less.

When taken, the anti-HCoV composition according to one or more embodiments of the present invention can act against HCoVs, reduce HCoV proliferation in non-immune cells, prevent HCoV infections, prevent the onset of HCoV infectious disease, prevent the aggravation of symptoms of HCoV infectious disease, and allow quick recovery from HCoV infectious disease onset. The specific form of an intake may be ingestion.

The anti-HCoV composition according to one or more embodiments of the present invention may be used as a preventive of virus infectious disease before the onset or as a curative after the onset. The anti-HCoV composition may instead be used to prevent the onset of suspected infections. In the working example implemented by the present inventors, after being added to target cells, an exopolysaccharide produced from lactic acid bacteria in the genus *Lactobacillus* exhibited its effects in an early stage. Thus, when immediately taken at the occurrence of suspected infections, the anti-HCoV composition according to one or more embodiments of the present invention may be expected to reduce HCoV proliferation at the early stage of infection to reduce the onset, or mitigate symptoms after the infection and the onset.

The anti-HCoV composition according to one or more embodiments of the present invention may indicate information including the use purpose, effects, functions, active components, and method of use. Indication includes providing the information about the above effects to consumers. This indication may be any indication that reminds the consumers of the above information, and may include all the indications independent of the purpose of the indication, the details of the indication, an object or a medium on which the indication appears. For example, the indication includes indicating the above information on the packages or the containers of the products, indicating the information on the advertisement, price list, or relevant documents associated with the products and displaying or distributing the information, and providing the information electromagnetically (through the Internet).

A packaged product of the anti-HCoV composition according to one or more embodiments of the present invention may have an indication of, for example, anti-HCoV, HCoV proliferation reduction, HCoV proliferation reduction in non-immune cells, HCoV infection preventive, HCoV infectious disease onset preventive, HCoV infectious disease aggravation preventive, or quick recovery from HCoV infectious disease onset.

The wording used for the above indication is not limited to the above examples, and may be another wording synonymous with the above wording. Examples of wordings usable for the consumers include enhance resistance to HCoV, retain resistance to HCoV, not lower resistance to HCoV, reduce HCoV proliferation, reduce HCoV proliferation in non-immune cells, prevent HCoV infections, prevent onset of HCoV infections, prevent aggravation of symptoms of HCoV infections, and quickly recover body from HCoV infectious disease onset.

The above anti-HCoV composition may be in the form of special purpose foods, comprehensively nutritious food, nutritional supplements, designated health foods, foods with function claims, or processed foods. The anti-HCoV composition or an exopolysaccharide may be combined with a composition such as drinks other than yogurt drinks or liquid food.

The embodiments of the present invention will be described in more detail based on a working example and a comparative example. The Working example described below is not intended to limit the present invention.

### Working Example 1

### 1. Preparation of Exopolysaccharide

*Lactobacillus delbrueckii subsp. bulgaricus* OLL1073R-1 was cultured for 18 hours at 37 °C on a medium of powdery skim milk of 10% by mass, and a culture was obtained. Trichloroacetic acid was then added to the culture to have a final concentration of 10% by mass, and then a denatured protein was removed from the culture. Cold ethanol was then added to the culture from which a denatured protein was removed, and then the culture was left still at 4 °C until and on the following day. A precipitate containing an exopolysaccharide (hereafter also referred to as EPS) was then obtained. Subsequently, the precipitate was dialyzed into Milli-Q water through a dialysis membrane (molecular cutoff of 6000 to 8000) to obtain dialysate. A nucleic acid and proteins in the dialysate underwent enzymolysis, and ethanol was added again to the dialysate to obtain a precipitate. The precipitate was dissolved into Milli-Q water and then dialyzed again, and then the dialysate was freeze-dried to obtain an intended EPS.

### 2. Anti-HCoV Test

### (1) Process of HCoV Infection on Human Fetal Lung Fibroblast Strain

The human fetal lung fibroblast strain MRC-5 was seeded on a culture flask of 25 cm², and cultured on an Eagle's minimal essential medium until becoming subconfluent. A culture fluid was then removed from the culture flask, the cell surface of the subconfluent human fetal lung fibroblast strain MRC-5 was washed with phosphate buffered saline once, and then a liquid containing HCoV (HCoV-229E) was inoculated on the human fetal lung fibroblast strain MRC-5 at a concentration having multiplicity of infection (MOI) of 0.1. After the inoculation of the liquid containing HCoV (HCoV-229E), HCoV (HCoV-229E) was cultured for one hour at 34 °C, the inoculated liquid containing HCoV (HCoV-229E) was removed, and the cell surface of the human fetal lung fibroblast strain MRC-5 was washed with phosphate buffered saline once. A liquid containing EPS of 200 pg/mL was then added by 5 mL into the human fetal lung fibroblast strain MRC-5, and the human fetal lung fibroblast strain MRC-5 was cultured at 34 °C. A cell culture supernatant was collected three, five, and seven days after the culture start date. For convenience, the cell culture supernatant three days after the culture start date is referred to as a three-day-old cell culture supernatant, the cell culture supernatant five days after the culture start date is referred to as a five-day-old cell culture supernatant, and a cell culture supernatant seven days after the culture start date is referred to as a seven-day-old cell culture supernatant.

### (2) HCoV Titer Measurement

First, the three-day-old cell culture supernatant, the five-day-old cell culture supernatant, and the seven-day-old cell culture supernatant were diluted serially to prepare various types of diluted solutions for each of the three-day-old cell culture supernatant, the five-day-old cell culture supernatant, and the seven-day-old cell culture supernatant. The human fetal lung fibroblast strain MRC-5 was seeded on a 96-well plate, and cultured until becoming subconfluent. The culture fluid was then removed from the 96-well plate, and the various types of diluted solutions for the three-day-old cell culture supernatant, the five-day-old cell culture supernatant, and the seven-day-old cell culture supernatant were inoculated on the human fetal lung fibroblast strain MRC-5 at N of 4. The human fetal lung fibroblast strain MRC-5 was then cultured at 34 °C. The human fetal lung fibroblast strain MRC-5 in the wells inoculated with the three-day-old cell culture supernatant, the human fetal lung fibroblast strain MRC-5 inoculated with the five-day-old cell culture supernatant, and the human fetal lung fibroblast strain MRC-5 inoculated with the seven-day-old cell culture supernatant were observed, and the wells at which cell degeneration was observed were counted. Thereafter, 50% tissue culture infectious dose (TCID50) was calculated with the Behrens-Karber method. The virus titer (TCID50/mL) was calculated from the 50% tissue culture infectious dose. The results are shown in FIG. 1. The results show that the human fetal lung fibroblast strain MRC-5 inoculated with the three-day-old cell culture supernatant and the human fetal lung fibroblast strain MRC-5 inoculated with the five-day-old cell culture supernatant have substantially the same virus titer, whereas the human fetal lung fibroblast strain MRC-5 inoculated with the seven-day-old cell culture supernatant has a virus titer about three times the virus titer of the human fetal lung fibroblast strain MRC-5 inoculated with the three-day-old cell culture supernatant or the human fetal lung fibroblast strain MRC-5 inoculated with the five-day-old cell culture supernatant.

### (Comparative Example 1)

In the same manner as in Working Example 1 except that an Eagle's minimal essential medium was used instead of the liquid containing EPS, the three-day-old cell culture supernatant, the five-day-old cell culture supernatant, and the seven-day-old cell culture supernatant were obtained, and the virus titer of the human fetal lung fibroblast strain MRC-5 was calculated in the same manner as in Working Example 1. The results are shown in FIG. 1. The results show that the human fetal lung fibroblast strain MRC-5 inoculated with the three-day-old cell culture supernatant and the human fetal lung fibroblast strain MRC-5 inoculated with the five-day-old cell culture supernatant have substantially the same titer, whereas the human fetal lung fibroblast strain MRC-5 inoculated with the seven-day-old cell culture supernatant has a virus titer about three times the virus titer of the human fetal lung fibroblast strain MRC-5 inoculated with the three-day-old cell culture supernatant or the human fetal lung fibroblast strain MRC-5 inoculated with the five-day-old cell culture supernatant. The virus titers are about three times higher than the virus titers in Working Example 1.

### (Overview)

Based on the results shown in Working Example 1 and Comparative Example, when EPS is added to the human fetal lung fibroblast strain MRC-5 infected with HCoV (HCoV-229E) and the human fetal lung fibroblast strain MRC-5 is cultured, the amount of HCoV (HCoV-229E) released into the culture supernatant of the human fetal lung fibroblast strain MRC-5 is reduced, or more specifically, proliferation of HCoV (HCoV-229E) is reduced. The human fetal lung fibroblast strain MRC-5 is a strain of non-immune cells, and has a weaker immune response to infection than immune cells. Thus, the virus proliferation reduction *in vivo* is expected to result largely from the function of the immune cells. However, EPS reduced the virus proliferation in the human fetal lung fibroblast strain MRC-5, which is a strain of non-immune cells. This is a notable result.

Coronaviruses generally invade biological cells, and proliferate while releasing RNA used to proliferate in the cells. A large amount of proliferated coronaviruses is further released from other cells also infected with coronaviruses, and the coronaviruses infect the other cells and the infection develops. The severity of the virus infectious disease is correlated with the amount of virus proliferated in the body. Reducing the virus proliferation in the cells can thus reduce the spread of infection between the cells, and is thus expected to prevent the onset of the coronavirus infection, prevent aggravation of the symptoms attributable to the coronavirus infection, or accelerate quick recovery from the onset of the HCoV infection.

A lung infectious disease such as pneumonia occurs when microorganisms including respiratory infectious disease viruses such as coronaviruses infect the lungs, proliferate, and damage the lungs. Thus, reducing proliferation of coronaviruses in lung cells is expected to prevent a lung infectious disease including pneumonia caused by coronaviruses.

### INDUSTRIAL APPLICABILITY

The anti-HCoV composition according to one or more embodiments of the present invention is a nonpharmaceutical composition that prevents HCoV infections, the onset of the infections, or aggravation of symptoms of the infections, and is expected to be effective on new HCoVs including MERS-CoV, SARS-CoV, and SARS-CoV2.

### Deposit Number

FERM BP-10741
FERM BP-10740

## Claims

1. An anti-human coronavirus composition, comprising:
an exopolysaccharide produced by a lactic acid bacterium in genus *Lactobacillus* as an active component.

2. The anti-human coronavirus composition according to claim 1, wherein the anti-human coronavirus composition is a composition to reduce proliferation of a human coronavirus.

3. The anti-human coronavirus composition according to claim 2, wherein the composition is a composition to reduce proliferation of a human coronavirus in non-immune cells.

4. The anti-human coronavirus composition according to claim 3, wherein the non-immune cells are lung cells.

5. The anti-human coronavirus composition according to claim 1, wherein the anti-human coronavirus composition is a preventive composition to prevent at least one selected from the group consisting of (a) a human coronavirus infection, (b) an onset of the human coronavirus infection, and (c) aggravation of a symptom attributable to the human coronavirus infection.

6. The anti-human coronavirus composition according to claim 1, wherein the anti-human coronavirus composition is a composition for quick recovery from an onset of a human coronavirus infection.

7. The anti-human coronavirus composition according to claim 1, wherein the human coronavirus is a respiratory infection human coronavirus.

8. The anti-human coronavirus composition according to claim 1, wherein the lactic acid bacterium in the genus *Lactobacillus* belongs to a *Lactobacillus delbrueckii* species.

9. The anti-human coronavirus composition according to claim 8, wherein the lactic acid bacterium in the genus *Lactobacillus* is *Lactobacillus delbrueckii subsp. bulgaricus* OLL1073R-1 (FERM BP-10741).
